# EUROPEAN PATENT APPLICATION

(11) **EP 3 868 356 A1**
(43) Date of publication of application: **25.08.2021**
(21) Application number: 19874246.2
(22) Date of filing: 17.10.2019
(51) Int. Cl.: A61K 8/02, A61K 8/34, A61K 8/35, A61K 8/73, A61K 8/81, A61Q 17/00, A61Q 19/00, B05B 5/00, B05B 5/04

(54) **METHOD FOR PRODUCING COATING FILM**

(30) Priority: 17.10.2018 JP 2018195880
(71) Applicant: Kao Corporation, Chuo-ku, Tokyo 103-8210 (JP)
(72) Inventor: ASAMI, Nobuyuki, Tokyo 131-8501 (JP); OKADA, Tomonari, Tokyo 131-8501 (JP); KAWAKAMI, Naoya, Tokyo 131-8501 (JP); OZAWA, Satoshi, Odawara-shi, Kanagawa 250-0002 (JP)
(74) Representative: Hoffmann Eitle
(86) International application number: PCT/JP2019/040785
(87) International publication number: WO 2020/080449

(57) **Abstract**

Provided is a method for producing a coating film on the skin by electrostatic spraying, the coating film having excellent appearance and excellent scratch resistance and stretch resistance.

A method for producing a coating film on skin, comprising the steps of:
A) electrostatically spraying a composition X comprising a component (a) and a component (b) directly to the skin to form a coating film on a surface of the skin:
(a) one or more volatile substances selected from the group consisting of water, an alcohol and a ketone;
(b) a film-forming polymer; and

B) applying a composition Y comprising a component (c) and a component (d) to the skin:
(c) 0.3% by mass or more and 10% by mass or less of a water-soluble polymer;
(d) 40% by mass or more and 95% by mass or less of water

in the order presented or in reverse order.

## Description

### Field of the Invention

The present invention relates to a method for producing a coating film.

### Background of the Invention

A method of forming a coating film on skin by electrostatic spraying has been reported. For example, Patent Literature 1 discloses a method for treating skin including electrostatically spraying the skin with a composition. The composition used in the method contains a liquid-insulating material, a conductive material, a particulate powder material, and a thickener.

Typically, a cosmetic product containing a pigment or a skincare composition is used as the composition. Specifically, a cosmetic foundation is used as the composition. That is, the inventions disclosed in Patent Literature 1 are primarily envisioned for cosmetic purposes by electrostatically spraying a cosmetic foundation to cosmetically decorate the skin. Also described in Patent Literature 2 is a disposable cartridge for use in an electrostatic spraying apparatus for cosmetics.

However, it was found that when an electrostatic spraying is performed according to the methods described in Patent Literatures 1 and 2 to form a coating on the skin, the adhesion between the skin and the coating formed by the electrostatic spraying is not sufficient, and the coating may be damaged or peeled off due to an external force such as friction. Accordingly, the present inventors found that, when a liquid agent containing water, a polyol or an oil of a liquid at 20°C, or a cosmetic containing 10 mass % or more of a solid oil at 20°C is applied, before or after forming a coating by electrostatic spraying on the skin, adhesion of the coating obtained by electrostatic spraying is improved, and thus, and the applicant filed patent applications (Patent Literatures 3 and 4).

### Citations

### Patent Literatures

(Patent Literature 1) JP-A-2006-104211
(Patent Literature 2) JP-A-2003-507165
(Patent Literature 3) JP-A-2017-78062
(Patent Literature 4) JP-B-6,316,495

### Summary of the Invention

That is, the present invention provides a method for producing a coating film on skin, the method comprising the steps of:
A) electrostatically spraying a composition X comprising a component (a) and a component (b) directly to the skin to form a coating film on a surface of the skin:
   (a) one or more volatile substances selected from the group consisting of water, an alcohol and a ketone;
   (b) a film-forming polymer; and
B) applying a composition Y comprising a component (c) and a component (d) to the skin:
   (c) 0.3% by mass or more and 10% by mass or less of a water-soluble polymer;
   (d) 40% by mass or more and 95% by mass or less of water
in the order presented or in reverse order.

The present invention also provides a composition Y comprising a component (c) and a component (d), the composition Y being used to be applied to skin in a manner other than electrostatic spraying to produce a coating film on the skin before or after forming a coating film on a surface of the skin by direct electrostatic spraying to the skin:
(c) 0.3% by mass or more and 10% by mass or less of a water-soluble polymer;
(d) 40% by mass or more and 95% by mass or less of water.

### Advantageous Effect of the Invention

According to the method for producing a coating film of the present invention, the coating film formed on the skin by electrostatic spraying has excellent appearance, and the coating film is excellent in adhesion and durability without breaking or floating due to scraping by a finger or stretching of the skin.

### Brief Description of the Drawings

Fig. 1 shows a schematic diagram of a configuration of an electrostatic spraying apparatus suitably used in the present invention.
Fig.2 shows a schematic diagram showing a state of performing an electrostatic spraying method using an electrostatic spraying apparatus.

### Description of Embodiments

The coating film on the skin obtained by the method disclosed in Patent Literatures 3 and 4 has excellent adhesiveness to the skin, but we have found that the film sometimes peels off or floats due to scratching with the finger, or expansion and contraction of the skin, and the transparency of the film also needs to be further improved.

Accordingly, the present invention provides a method for producing a coating film having excellent appearance and excellent scratch resistance and stretch resistance, formed on the skin by electrostatic spraying.

Then, the present inventors conducted various studies on components of the composition to be applied before or after electrostatic spraying to the skin. First, the inventors considered application of a composition containing water in order to obtain a coating film with a fresh touch, failing in obtaining the desired coating film. Then the inventors found that a coating film having excellent transparency, excellent scratch resistance and stretch resistance and fresh feeling can be formed by using a composition containing a small amount of a water-soluble polymer and a large amount of water, to complete the present invention.

The present invention includes the steps of A) forming a coating film on a surface of skin by electrostatically spraying a composition X directly to the skin (Step A)); and B) applying a composition Y to the skin (Step B)).

An electrostatic spraying method is used in the present invention as a method for forming a coating film in the Step A). In the electrostatic spraying method, positive or negative high voltage is applied to a composition so that the composition is charged, and the composition which has been charged is sprayed to the target. While particles of the composition sprayed are repeatedly made smaller due to the Coulomb repulsion, the composition spreads into air, and as the solvent, which is a volatile substance, is dried during that process, or after being attached to the target, a coating film is formed on the surface of the target.

The composition X used in the present invention (hereinafter the composition X is also referred to as "spraying composition") is liquid in an environment in which an electrostatic spraying method is performed. The composition X includes the following component (a) and component (b):
(a) one or more volatile substances selected from the group consisting of water, an alcohol and a ketone;
(b) a film-forming polymer.

In the following the respective components will be described.

The volatile substance as the component (a) is volatile in the liquid state. The component (a) is compounded in the spraying composition for the purpose of forming coating film on the skin; the spraying composition placed in an electric field is thoroughly charged and then discharged to the skin from the tip of a nozzle, and as the component (a) evaporates, the charge density of the spraying composition becomes excessively high, and while particles of the composition are made even smaller due to the Coulomb repulsion, the component (a) further evaporates and consequently a dry coating film is formed on the skin. To this end, the volatile substance has a vapor pressure at 20°C of preferably 0.01 kPa or more and 106.66 kPa or less, more preferably 0.13 kPa or more and 66.66 kPa or less, further preferably 0.67 kPa or more and 40.00 kPa or less, and still more preferably 1.33 kPa or more and 40.00 kPa or less.

A monovalent linear aliphatic alcohol, a monovalent cycloaliphatic alcohol and a monovalent aromatic alcohol, for example, are preferably used as the alcohol among the volatile substances as the component (a). Examples of the monovalent linear aliphatic alcohol may include alcohols having 1 to 6 carbon atoms, examples of the monovalent cycloaliphatic alcohol may include cyclic alcohols having 4 to 6 carbon atoms and examples of the monovalent aromatic alcohol may include benzyl alcohol and phenylethyl alcohol. Specific examples thereof may include ethanol, isopropyl alcohol, butyl alcohol, phenylethyl alcohol, n-propanol and n-pentanol. One or more selected from the group consisting of these alcohols may be used.

Examples of the ketones among the volatile substances as the component (a) may include a dialkylketone having 1 to 4 carbon atoms such as acetone, methyl ethyl ketone and methyl isobutyl ketone. These ketones may be used singly or in combinations of two or more.

The volatile substance as the component (a) is more preferably one or more selected from the group consisting of ethanol, isopropyl alcohol, butyl alcohol and water, further preferably one or more selected from the group consisting of ethanol and butyl alcohol, and still more preferably a volatile substance containing at least ethanol.

The content of the component (a) in the spraying composition is preferably 30% by mass or more, more preferably 55% by mass or more, and further preferably 60% by mass or more. The content is preferably 98% by mass or less, more preferably 96% by mass or less, and further preferably 94% by mass or less. The content of the component (a) in the spraying composition is preferably 30% by mass or more and 98% by mass or less, more preferably 55% by mass or more and 96% by mass or less, and further preferably 60% by mass or more and 94% by mass or less. When the spraying composition contains the component (a) in this ratio, the spraying composition can be thoroughly evaporated when an electrostatic spraying method is performed.

The content of ethanol is preferably 50% by mass or more, more preferably 65% by mass or more, further preferably 80% by mass or more based on the total amount of the volatile substance as the component (a). The content is preferably 100% by mass or less. The content of ethanol is preferably 50% by mass or more and 100% by mass or less, more preferably 65% by mass or more and 100% by mass or less, and further preferably 80% by mass or more and 100% by mass or less based on the total amount of the volatile substance as the component (a).

From the viewpoint of fiber formability and conductivity, the content of water is preferably less than 50% by mass, more preferably 45% by mass or less, further preferably 10% by mass or less, and still more preferably 5% by mass or less, and preferably 0.2% by mass or more, and more preferably 0.4% by mass or more based on the total amount of the volatile substance as the component (a).

The film-forming polymer, which is the component (b), is usually soluble in the volatile substance as the component (a). Here, being soluble means that the polymer is in the form of dispersion at 20°C and the dispersion is homogeneous, and preferably transparent or semi-transparent when visually observed.

A suitable polymer is used as the film-forming polymer in accordance with the properties of the volatile substance as the component (a). More specifically, the film-forming polymer is roughly classified into water-soluble polymers and water-insoluble polymers. In the present description, the "water-soluble polymer" refers to a polymer with such a characteristic that when 1 g of the polymer is weighed in an environment of 1 atm and 23°C and then dipped in 10 g of ion exchange water for 24 hours, 0.5 g or more of the polymer which has been immersed dissolves in water. Meanwhile, the "water-insoluble polymer" in the present description refers to a polymer with such a characteristic that when 1 g of the polymer is weighed in an environment of 1 atm and 23°C and then dipped in 10 g of ion exchange water for 24 hours, 0.5 g or more of the polymer which has been immersed does not dissolve in water.

Examples of the water-soluble polymers capable of forming a coating film may include mucopolysaccharides such as pullulan, hyaluronic acid, chondroitin sulfate, poly-γ-glutamic acid, modified corn starch, β-glucan, glucooligosaccharide, heparin and keratosulfate, natural polymers such as cellulose, pectin, xylan, lignin, glucomannan, galacturonic acid, psyllium seed gum, tamarind seed gum, gum arabic, gum traganth, water-soluble soybean polysaccharide, alginic acid, carrageenan, laminaran, agar (agarose), fucoidan, methyl cellulose, hydroxypropyl cellulose and hydroxypropyl methyl cellulose; and synthetic polymers such as partially saponified polyvinyl alcohol (when not used in combination with a cross-linking agent), low saponified polyvinyl alcohol, polyvinyl pyrrolidone (PVP), polyethylene oxide and sodium polyacrylate. These water-soluble polymers may be used singly or in combinations of two or more. Of these water-soluble polymers, pullulan and synthetic polymers such as partially saponified polyvinyl alcohol, low saponified polyvinyl alcohol, polyvinyl pyrrolidone and polyethylene oxide are preferably used because a coating film is easily produced. When polyethylene oxide is used as the water-soluble polymer, polyethylene oxide has a number average molecular weight of preferably 50,000 or more and 3,000,000 or less, and more preferably 100,000 or more and 2,500,000 or less.

Examples of the water-insoluble polymers capable of forming a coating film may include completely saponified polyvinyl alcohol, which can be insolubilized after forming a coating film, partially saponified polyvinyl alcohol, which can be cross-linked after forming a coating film when used in combination with a cross-linking agent, an oxazoline-modified silicone such as a poly(N-propanoylethyleneimine)-grafted dimethylsiloxane/y-aminopropylmethylsiloxane copolymer, polyvinylacetal diethylaminoacetate, Zein (main component of corn protein), polyester, polylactic acid (PLA), an acrylic resin such as polyacrylonitrile resin and polymethacrylic acid resin, polystyrene resin, polyvinyl butyral resin, polyethylene terephthalate resin, polybutylene terephthalate resin, polyurethane resin, polyamide resin, polyimide resin and polyamideimide resin. These water-insoluble polymers may be used singly or in combinations of two or more. Of these water-insoluble polymers, one or more selected from the group consisting of completely saponified polyvinyl alcohol, which can be insolubilized after forming a coating film, partially saponified polyvinyl alcohol, which can be cross-linked after forming a coating film when used in combination with a cross-linking agent, polyvinyl butyral resin, polyurethane resin, an oxazoline-modified silicone such as a poly(N-propanoylethyleneimine)-grafted dimethylsiloxane/y-aminopropylmethylsiloxane copolymer, polyvinylacetal diethylaminoacetate and Zein are preferably used. One or more selected from the group consisting of polybutyral resin and polyurethane resin are more preferably used.

The content of the component (b) in the spraying composition is preferably 2% by mass or more, more preferably 4% by mass or more, and further preferably 6% by mass or more. The content is 50% by mass or less, more preferably 45% by mass or less, and further preferably 40% by mass or less. The content of the component (b) in the spraying composition is preferably 2% by mass or more and 50% by mass or less, more preferably 4% by mass or more and 45% by mass or less, and further preferably 6% by mass or more and 40% by mass or less. When the spraying composition contains the component (b) in this percentage, a coating film which is made of a deposit of fiber, covers the surface of the bear skin and has excellent durability over time with little smudging can be successfully formed.

The ratio of the contents of the component (a) and the component (b), ((a)/(b)), in the spraying composition is, from the viewpoint that the component (a) can be sufficiently volatilized when the electrostatic spraying method is carried out, preferably 0.5 or more and 40 or less, more preferably 1 or more and 30 or less and even more preferably 2 or more and 25 or less.

The ratio of ethanol and the content of the component (b), ((a)/(b)), in the spraying composition is, from the viewpoint that ethanol can be sufficiently volatilized when the electrostatic spraying method is carried out, preferably 0.5 or more and 40 or less, more preferably 1 or more and 30 or less and even more preferably 2 or more and 25 or less.

The spraying composition can comprise a glycol. The glycol includes ethylene glycol, propylene glycol, butylene glycol, diethylene glycol, dipropylene glycol and polyethylene glycol having a molecular weight of 1000 or less. From the viewpoint that the component (a) can be sufficiently volatilized when the electrostatic spraying method is carried out, the glycol is, in the spraying composition, preferably 10% by mass or less, more preferably 3% by mass or less, and even more preferably 1% by mass or less.

The spraying composition can further contain a powder. The powder includes coloring pigments, extender pigments, pearl pigments and organic powders. The powder is, from the viewpoint of imparting smooth feel to skin surfaces, in the spraying composition, preferably 5% by mass or less, more preferably 3% by mass or less and even more preferably 1% by mass or less, and it is preferable to contain substantially no powder.

The spraying composition may contain only the above- mentioned component (a) and component (b), or may contain, in addition to the component (a) and component (b), other components. Examples of the other components include oils such as di(phytosteryl/octyldodecyl) lauroylglutamate ans silicone oil, surfactants, UV protective agents, fragrances, repellants, antioxidants, stabilizers, antiseptics, antiperspirants and various vitamins. Here, these agents are not limited to their original applications as the agents, but can be used for other applications according to purposes, for example, an antiperspirant can be used as a fragrance. Alternatively, these agents can be used as having multiple purposes; for example, an antiperspirant can also serve as a fragrance. Where the spraying composition contains the other components, the content rate of the other components is preferably 0.1% by mass or more and 30% by mass or less and more preferably 0.5% by mass or more and 20% by mass or less.

In the method of the present invention, before or after the step B, a coating is formed directly on a skin surface by electrostatically spraying the spraying composition.

When the electrostatic spraying method is carried out, preferably used is a spraying composition whose viscosity at 25°C is 1 mPa·s or more, more preferably 10 mPa·s or more and even more preferably 50 mPa·s or more. Also preferably used is a spraying composition whose viscosity at 25°C is 5,000 mPa·s or less, more preferably 2,000 mPa·s or less and even more preferably 1,500 mPa·s or less. The viscosity at 25°C of the spraying composition is preferably 1 mPa·s or more and 5,000 mPa·s or less, more preferably 10 mPa·s or more and 2,000 mPa·s or less and even more preferably 50 mPa·s or more and 1,500 mPa·s or less. Use of the spraying composition having a viscosity in this range successfully enables formation of a coating, particularly a porous coating composed of a deposit of fiber, by the electrostatic spraying method. The formation of the porous coating is advantageous from the viewpoint of improving prevention of stuffiness of skin, from the viewpoint of improving adhesiveness of the coating to skin, from the viewpoint that the coating, when peeled off the skin, can be peeled off easily and cleanly, and from other viewpoints. The viscosity of the spraying composition is measured at 25°C by using an E-type viscometer. As the E-type viscometer, for example, an E-type viscometer, manufactured by Tokyo Keiki Inc. can be used. As a rotor in this case, a rotor No. 43 can be used.

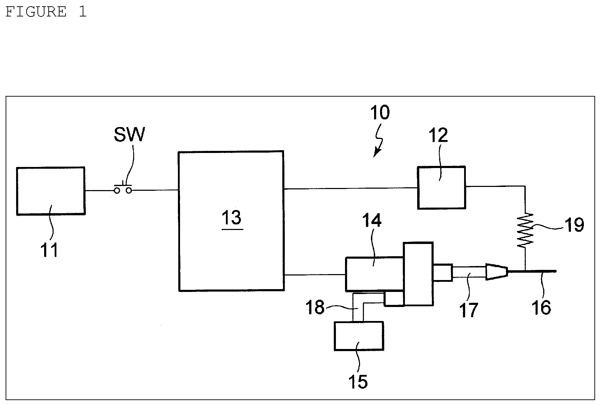

The spraying composition is sprayed directly to a skin of a human by the electrostatic spraying method. The electrostatic spraying method includes, in an electrostatic spraying step, a step of electrostatically spraying the spraying composition on a skin by using an electrostatic spraying apparatus to thereby form a coating. The electrostatic spraying apparatus has a container accommodating the spraying composition, a nozzle for discharging the spraying composition, a supply device for supplying the spraying composition accommodated in the container to the nozzle, and a power source for applying a voltage to the nozzle. Figure 1 shows a schematic diagram indicating an electrostatic spraying apparatus suitably used in the present invention. An electrostatic spraying apparatus 10 shown in Figure 1 has a low-voltage power source 11. The low-voltage power source 11 is one which can generate a voltage of several volts to several tens of volts. For the purpose of enhancing transportability of the electrostatic spraying apparatus 10, it is preferable that the low-voltage power source 11 be composed of one or more batteries. Use of batteries as the low-voltage power source 11 exhibits also such an advantage that the batteries can be exchanged easily as required. In place of the batteries, an AC adaptor or the like can be used as the low-voltage power source 11.

The electrostatic spraying apparatus 10 has also a high-voltage power source 12. The high-voltage power source 12 is connected to the low-voltage power source 11, and has an electronic circuit (not shown in figure) for boosting a voltage generated by the low-voltage power source 11 to a high voltage. The boosting electronic circuit is usually constituted of a transformer, capacitors, semiconductor elements and the like.

The electrostatic spraying apparatus 10 further has an auxiliary electric circuit 13. The auxiliary electric circuit 13 intervenes between the above-mentioned low-voltage power source 11 and high-voltage power source 12, and has a function of regulating the voltage of the low-voltage power source 11 to cause the high-voltage power source 12 to stably operate. The auxiliary electric circuit 13 further has a function of controlling the rotation frequency of a motor equipped with a pump mechanism 14 described later. The control of the rotation frequency of the motor leads to control of the amount of the spraying composition supplied from a container 15 described later of the spraying composition to the pump mechanism 14. A switch SW is installed between the auxiliary electric circuit 13 and the low-voltage power source 11, and is so configured that the electrostatic spraying apparatus 10 can be operated/stopped by on/off of the switch SW.

The electrostatic spraying apparatus 10 further has a nozzle 16. The nozzle 16 is composed of various electroconductors including metals and non-electroconductors such as plastics, rubbers and ceramics, and has a shape which can discharge the spraying composition from its tip. In the nozzle 16, a fine space through which the spraying composition flows is formed along the longitudinal direction of the nozzle 16. It is preferable that the size of the fine space for the size of the cross section be 100 µm or more and 1,000 µm or less in diameter. The nozzle 16 communicates with the pump mechanism 14 through a pipe 17. The pipe 17 may be an electroconductor or a non-electroconductor. Then, the nozzle 16 is electrically connected to the high-voltage power source 12. Thereby, the nozzle 16 is so configured that a high voltage can be applied thereto. In this case, in order to prevent an excessive current from flowing in a human body when the human body touches directly the nozzle 16, the nozzle 16 and the high-voltage power source 12 are electrically connected through a current limiting resistance 19.

The pump mechanism 14 communicating with the nozzle 16 through the pipe 17 functions as a supply device for supplying the pump mechanism 14 stored in the container 15 to the nozzle 16. The pump mechanism 14 operates by receiving a power from the low-voltage power source 11. The pump mechanism 14 is constituted so as to supply a predetermined amount of the spraying composition to the nozzle 16 under control by the auxiliary electric circuit 13.

To the pump mechanism 14, the container 15 is connected through a flexible pipe 18. The spraying composition is accommodated in the container 15. It is preferable that the pump mechanism 14 be of a gear pump type or a piston pump type. It is preferable that the container 15 have a cartridge-type exchangeable form.

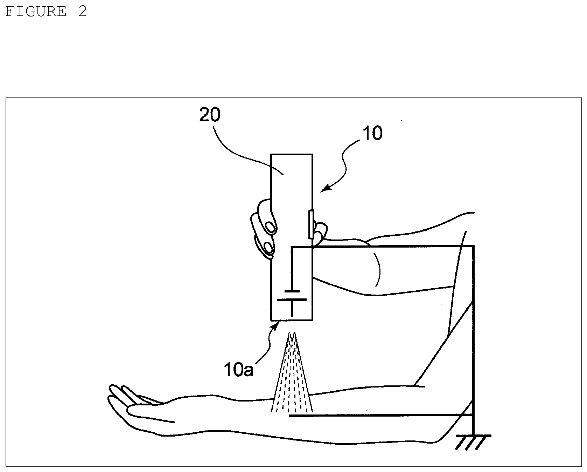

The electrostatic spraying apparatus 10 having the above constitution can be used, for example, as shown in Figure 2. Figure 2 shows a hand-held type electrostatic spraying apparatus 10 having a size holdable by one hand. The electrostatic spraying apparatus 10 shown in the figure is so configured that all members of the schematic diagram shown in Figure 1 are accommodated in a cylindrical housing 20. On one end 10a in the longitudinal direction of the housing 20, a nozzle (not shown in figure) is disposed. The nozzle is arranged on the housing 20 so as to protrude toward a skin side as a coating forming target so that the jetting direction of the composition is made to coincide with the longitudinal direction of the housing 20. By arranging the nozzle so that the nozzle tip protrudes toward a coating forming target in the longitudinal direction of the housing 20, it becomes hard for the spraying composition to adhere on the housing and a coating can be formed stably.

Where a skin as the coating forming target is a user's own skin, when an electrostatic spraying apparatus 10 is operated, the user, that is, a person forming a coating on the person's own skin by electrostatic spraying, holds the apparatus 10 by hand and directs one end 10a of the apparatus 10 having a nozzle (not shown in figure) arranged thereon toward a target site of electrostatic spraying. Figure 2 illustrates a state that the one end 10a of the electrostatic spraying apparatus 10 is directed to an inner side of the user's forearm. Under this state, a switch of the apparatus 10 is turned on to carry out the electrostatic spraying method. A power is supplied to the apparatus 10 to generate an electric field between the nozzle and the skin. In an embodiment illustrated in Figure 2, a high positive voltage is applied to the nozzle and the skin becomes a negative electrode. When the electric field is generated between the nozzle and the skin, the spraying composition on the nozzle tip is depolarized by electrostatic induction and the frontend portion of the spraying composition spreads to a cone-like shape; and charged droplets of the spraying composition are discharged from the spread cone frontend along the electric field into the air toward the skin. As the component (a) serving as a solvent evaporates from the charged spraying composition discharged into the space and, the charge density of the surface of the spraying composition becomes excessive, so that while micronization of the spraying composition is repeated by the Coulomb repellency, the spraying composition spreads into the space and reaches the skin. In this case, by suitably regulating the viscosity of the spraying composition, the sprayed spraying composition can be made to reach the skin in the state of being droplets. Alternatively, it is also possible that during the discharge into the space, the component (a) of a volatile substance serving as a solvent is caused to volatilize from the composition to cause a polymer serving as a solute having film-forming ability to solidify, the spraying composition is caused to extensionally deform due to a potential difference and to form fibers, which deposit on the surface of the skin. For example, when the viscosity of the spraying composition is raised, it becomes easy for the composition to be deposited in a form of fiber on the skin surface. Thereby, a coating composed of a deposit of the fiber is formed on the skin surface. A coating composed of a deposit of fiber can be also formed by adjusting the distance between the nozzle and the skin or the voltage to be applied to the nozzle.

During carrying out of the electrostatic spraying method, a high potential difference is generated between the skin which is a coating forming target and the nozzle. Since the impedance is very high, however, the current flowing in the human body is remarkably minute. The present inventors have confirmed that the current flowing in a human body during carrying out of the electrostatic spraying method is lower by several digits than, for example, the current flowing in human bodies due to a static electricity generated in usual lives.

In forming a deposit of fiber by the electrostatic spraying method, the thickness of the fibers is, in terms of equivalent circle diameter, preferably 10 nm or more and more preferably 50 nm or more. Then, the thickness is preferably 3,000 nm or less and more preferably 2,000 nm or less. The thickness of the fibers can be measured, for example, by observing the fibers in a magnification of 10,000X by a scanning electron microscope (SEM) observation, arbitrarily choosing 10 fibers excluding defects (lumps of fibers, crossing portions of fibers, droplets) from its two-dimensional image, drawing lines orthogonal to the longitudinal directions of the fibers, and directly reading the fiber diameters.

The above fiber, under the principle of its production, becomes an infinite-length continuous fiber, but it is preferable that the fiber have a length at least 100 or more times a thickness of the fiber. In the present description, a fiber having a length 100 or more times a thickness of the fiber is defined as a "continuous fiber". Then, it is preferable that a coating produced by the electrostatic spraying method be a porous discontinuous coating composed of a deposit of continuous fiber. The coating of such a form not only can be handled as one sheet as an aggregate, but also has a feature of being very soft, and has advantages of not falling into pieces even under a shearing force and excellence in followability to movements of the body. The coating also has an advantage of excellence in diffusibility of sweat generated from skin. The coating further also has an advantage of being easily peeled off. By contrast, a continuous coating having no pores is not easily peeled off and since the diffusibility of sweat is very low, easily causes stuffiness on skin.

The spraying composition having become fibrous reaches a skin, in a charged state. Since the skin is also charged as described before, the fibers adhere closely with the skin by an electrostatic force.
Since fine irregularities such as texture are present on the surface of skin, the fibers adhere more closely with the skin surface conjointly with the anchor effect by the irregularities. When the electrostatic spraying is thus completed, the power source of the electrostatic spraying apparatus 10 is turned off. Thereby, the electric field between the nozzle and the skin vanishes and charges are immobilized on the skin surface. Consequently, the adhesiveness of the coating further develops.

Although the above description has been for a porous coating composed of a deposit of fiber as the coating, forms of coatings are not limited thereto; a continuous coating having no pores may be formed; or a porous coating having a form other than a deposit of fiber, for example, a porous coating made by irregularly or regularly forming a plurality of throughholes on a continuous coating, that is, a discontinuous coating may be formed. As described above, by controlling the viscosity of the spraying composition, the distance between the nozzle and a skin, the voltage applied to the nozzle, and the like, a coating having an optional shape can be formed.

It is preferable, for successfully forming a coating, that the distance between the nozzle and a skin is, though depending also on the voltage applied to the nozzle, 50 mm or more and 150 mm or less. The distance between the nozzle and a skin can be measured by a noncontact type sensor usually used, or the like.

Irrespective of whether or not a coating formed by the electrostatic spraying method is porous, the basis weight of the coating is preferably 0.1 g/m² or more and more preferably 1 g/m² or more. Then, the basis weight is preferably 30 g/m² or less and more preferably 20 g/m² or less. The basis weight of the coating is, for example, preferably 0.1 g/m² or more and 30 g/m² or less and more preferably 1 g/m² or more and 20 g/m² or less. By thus setting the basis weight of a coating, the adhesiveness of the coating can be improved. Then, the electrostatic spray step of electrostatically spraying the composition directly on a skin to thereby form a coating means a step of carrying out electrostatic spraying on a skin to thereby form a coating. A step of electrostatically spraying a composition on a place other than a skin to form a sheet composed of fibers, and applying the sheet to a skin surface is different from the above electrostatic spray step.

Then, the step B) will be described.

The step B) is a step of applying, to the skin, the composition Y, other than the composition X (spraying composition), comprising one or more selected from the group consisting of a component (c) and a component (d). The step B) is carried out before the step elestrostatic spraying step A).
(c) 0.3% by mass or more and 10% by mass or less of a water-soluble polymer
(d) 40% by mass or more and 95% by mass or less of water.

The step B) is a step of applying the composition Y to a skin surface by using a unit other than electrostatic spraying.

The component (c), a water-soluble polymer, used for the composition Y contributes to improvement of transparency and improvement of scratch resistance and stretch resistance of the coating film formed on the skin by electrostatic spraying. The water-soluble polymer (c) may be a polymer with such a characteristic that when 1 g of the polymer is weighed in an environment of 1 atm and 23°C and then dipped in 10 g of ion exchange water for 24 hours, 0.5 g or more of the polymer which has been immersed dissolves in water. Examples thereof include plant polymers, microbial polymers, animal polymers, alginic acid polymers, mucopolysaccharides, cellulose polymers, starch polymers, vinyl polymers, acrylic polymers and polyoxyethylene polymers.

Examples of the plant polymers may include gum arabic, tragacanth, arabinogalactan, locust bean gum, tara gum, guar gum, fenugreek gum, karaya gum, carrageenan, pectin, agar, quince seed (marmelo), starch (rice, corn, potato, wheat), algae colloid and trant gum.

Examples of the microbial polymers may include xanthan gum, dextran, succinoglucan and pullulan.

Examples of the animal polymers may include collagen, casein, albumin, deoxyribonucleic acid (DNA) and a salt thereof.

Examples of the alginic acid polymers may include sodium alginate and propylene glycol alginate.

Examples of the mucopolysaccharides may include hyaluronic acid, tuberose polysaccharide and tremella fuciformis polysaccharide.

Examples of the cellulose polymers may include methyl cellulose, ethyl cellulose, methyl hydroxypropyl cellulose, carboxymethyl cellulose, hydroxymethyl cellulose, hydroxyethyl cellulose, hydroxypropyl cellulose, nitrocellulose, sodium cellulose sulfate, sodium carboxymethyl cellulose, crystalline cellulose, cellulose powder and sodium hydroxyethylcellulose hydroxypropyl stearyl ether hydroxypropylsulfonate.

Examples of the starch polymers may include carboxymethyl starch and methyl hydroxypropyl starch.

Examples of the vinyl polymers may include polyvinyl methyl ether, polyvinyl pyrrolidone and carboxyvinyl polymer.

Examples of the acrylic polymers may include sodium polyacrylate, polyethylacrylate, polyacrylic acid amide, alkyl acrylate-methacrylate copolymer, (acrylate/alkyl acrylate (C10-30)) crosspolymer, (Na acrylate/acryloyldimethyltaurine Na) copolymer and polyacrylate crosspolymer-6.

Examples of the polyoxyethylene polymers may include polyethylene glycol, polyethylene glycol silane and polyoxyethylene polyoxypropylene copolymer.

One or more of the water-soluble polymers may be used.

Of these water-soluble polymers, one or more selected from the group consisting of carrageenan, agar, xanthan gum, pullulan, hyaluronic acid, tuberose polysaccharide (TPS), carboxymethyl cellulose, hydroxyethyl cellulose, sodium hydroxyethylcellulose hydroxypropyl stearyl ether hydroxypropylsulfonate, polyvinyl pyrrolidone, carboxyvinyl polymer, (acrylate/alkyl acrylate (C10-30)) crosspolymer, (Na acrylate/acryloyldimethyltaurine Na) copolymer and polyacrylate crosspolymer-6 are preferred from the viewpoint of improvement of scratch resistance and stretch resistance and improvement of transparency.

The content of the water-soluble polymer (c) in the composition Y is 0.3% by mass or more and 10% by mass or less from the viewpoint of transparency of the coating film and scratch resistance and stretch resistance of the coating film. The content is preferably 0.4% by mass or more, more preferably 0.5% by mass or more. Furthermore, the content is preferably 8% by mass or less, more preferably 7% by mass or less, and further preferably 6% by mass or less. More specifically, the content is preferably 0.4% by mass or more and 8% by mass or less, more preferably 0.4% by mass or more and 7% by mass or less, and further preferably 0.5% by mass or more and 6% by mass or less.

The component (d) used for the composition Y is water. When a solution in which a water-soluble polymer (e) is dissolved in a large amount of water (d) (composition Y) is applied to the skin before or after the Step A), transparency, scratch resistance and stretch resistance of the coating film formed are improved. The content of water (d) in the composition Y is 40% by mass or more and 95% by mass or less from the viewpoint of improvement of transparency and improvement of scratch resistance and stretch resistance of the coating film. The content is preferably 50% by mass or more, more preferably 60% by mass or more, and further preferably 70% by mass or more. The content is preferably 94.8% by mass or less, more preferably 94.5% by mass or less, and further preferably 94.0% by mass or less. More specifically, the content is preferably 50% by mass or more and 94.8% by mass or less, more preferably 60% by mass or more and 94.5% by mass or less, and further preferably 70% by mass or more and 94.0% by mass or less.

The mass ratio of the component (d) to the component (c) in the composition Y, ((d)/(c)), is preferably 10 or more, more preferably 15 or more, and further preferably 20 or more, and preferably 400 or less, more preferably 300 or less, and further preferably 250 or less from the viewpoint of improvement of transparency, scratch resistance and stretch resistance of the coating film, and in order to obtain fresh feeling. More specifically, the mass ratio is preferably 10 or more and 400 or less, more preferably 15 or more and 300 or less, and further preferably 20 or more and 250 or less.

It is preferable that the composition Y further comprise a component (e), a polyol, from the viewpoint of improving transparency, scratch resistance and stretch resistance of the coating film.

Examples of the polyol may include alkylene glycols such as ethylene glycol, propylene glycol, 1,3-propanediol and 1,3-butanediol; polyalkylene glycols such as diethylene glycol, dipropylene glycol, polyethylene glycol having a molecular weight of 1,000 or less and polypropylene glycol; and glycerols such as glycerol, diglycerol and triglycerol. Of them, ethylene glycol, propylene glycol, 1,3-butanediol, dipropylene glycol, polyethylene glycol having a molecular weight of 1,000 or less, glycerol and diglycerol are preferred, and propylene glycol, 1,3-butanediol and glycerol are more preferred from the viewpoint of improving transparency, scratch resistance and stretch resistance of the coating film. It is further preferable that the polyol (e) include at least glycerol.

The content of the component (e) in the composition Y is preferably 1% by mass or more, more preferably 2% by mass or more, and further preferably 3% by mass or more from the viewpoint of improving transparency, scratch resistance and stretch resistance of the coating film. The content thereof is preferably 30% by mass or less, more preferably 25% by mass or less, and further preferably 20% by mass or less. More specifically, the content thereof is preferably 1% by mass or more and 30% by mass or less, more preferably 2% by mass or more and 25% by mass or less, and further preferably 3% by mass or more and 20% by mass or less.

The mass ratio of the component (e) to the component (c) in the composition Y, ((e)/(c)), is preferably 0.3 or more, more preferably 0.5 or more, and further preferably 1 or more from the viewpoint of improving scratch resistance and stretch resistance of the coating film. The mass ratio is preferably 100 or less, more preferably 40 or less, and further preferably 14 or less. More specifically, the mass ratio is preferably 3 or more and 100 or less, more preferably 0.5 or more and 40 or less, and further preferably 1 or more and 14 or less.

The composition Y may also contain a surfactant, a small amount of an oil agent, an antioxidant, a flavoring agent, a coloring, an antiseptic, a pH adjuster, a blood circulation promoter, a cooling agent, an antiperspirant, a disinfectant, a skin activator, a moisturizer, a refrigerant and the like in addition to the above components. Examples of the surfactant may include a nonionic surfactant, a cationic surfactant, an anionic surfactant and an amphoteric surfactant. The form of the composition Y may be an aqueous solution or an oil-in-water emulsion composition. The content of the oil agent in the composition Y is preferably 5% by mass or less, more preferably 3% by mass or less, and further preferably 1% by mass or less from the viewpoint of fresh feeling, transparency and the like of the coating film.

The step of applying the composition Y to the skin (Step B)) may be performed before or after the Step A). The manner for applying the composition Y to the skin is not specifically limited as long as it is other than the electrostatic spraying. Examples thereof may include applying it to the skin with fingers and applying it to the skin using an applicator.

Makeup may also be applied to the skin before, between or after the Step A) and the Step B) by applying a cosmetic containing a powder to the skin (Step C)).
For example, the steps may be performed in the order of the Step A), the Step B) and the Step C), or the Step B), Step A) and the Step C). It is also preferable that the Step C) is performed in a manner other than electrostatic spraying.

Examples of the powder used for the cosmetic in the Step C) may include a color pigment, an extender, a pearl pigment and an organic powder. Examples of the color pigment may include an inorganic color pigment, an organic color pigment and an organic dye, and one or more thereof may be used.

Specific examples of the inorganic color pigment may include inorganic color pigments such as red iron oxide, iron hydroxide, iron titanate, yellow iron oxide, black iron oxide, carbon black, dark blue, ultramarine blue, dark blue titanium oxide, black titanium oxide, sintered products of titanium and titanium oxide, manganese violet, cobalt violet, chromium oxide, chromium hydroxide, cobalt oxide and cobalt titanate; and inorganic white pigments such as titanium oxide, zinc oxide, calamine, zirconium oxide, magnesium oxide, cerium oxide, aluminum oxide and a composite thereof. One or more thereof may be used.

Of them, at least one or more selected from the group consisting of iron oxide, titanium oxide and zinc oxide are preferred, and one or more selected from the group consisting of titanium oxide, zinc oxide, red iron oxide, yellow iron oxide and black iron oxide are more preferred.

Examples of the organic color pigment and organic dye may include organic tar pigments such as red No. 3, red No. 102, red No. 104, red No. 106, red No. 201, red No. 202, red No. 204, red No. 205, red No. 220, red No. 226, red No. 227, red No. 228, red No. 230, red No. 401, red No. 405, red No. 505, orange No. 203, orange No. 204, orange No. 205, yellow No. 4, yellow No. 5, yellow No. 401, blue No. 1 and blue No. 404; and organic dyes such as β-carotene, caramel, and paprika dyes. Those coated with a polymer such as cellulose or polymethacrylic acid ester may be used. It is preferable that of them, at least red No. 102 be included.

Examples of the extender may include barium sulfate, calcium sulfate, magnesium sulfate, magnesium carbonate, calcium carbonate, talc, mica, kaolin, sericite, silicic acid, silicic anhydride, aluminum silicate, magnesium silicate, magnesium aluminum silicate, calcium silicate, barium silicate, strontium silicate, metal tungstate, hydroxyapatite, vermiculite, clay, bentonite, montmorillonite, hectorite, smectite, zeolite, ceramic powder, dibasic calcium phosphate, alumina, silica, aluminum hydroxide, boron nitride, synthetic mica, synthetic sericite, metal soap and barium sulfate-treated mica. One or more thereof may be used.

It is preferable that of them, barium sulfate, calcium carbonate, mica, silicic anhydride, talc, boron nitride and synthetic mica be included.

Examples of the pearl pigment (glitter powder) may include fish foil, titanium oxide-coated mica (mica titanium), bismuth oxychloride, titanium oxide-coated bismuth oxychloride, titanium oxide-coated talc, titanium oxide-coated color mica, titanium oxide/iron oxide-coated mica, fine particulate titanium oxide-coated mica titanium, fine particulate zinc oxide-coated mica titanium, organic pigment-treated mica titanium, lower titanium oxide-coated mica, titanium oxide-coated synthetic mica, titanium oxide-coated plat-like silica, hollow plate-like titanium oxide, iron oxide-coated mica, plate-like iron oxide (MIO), aluminum flake, stainless flake, titanium oxide-coated plate-like alumina, glass flake, titanium oxide-coated glass flake, pearl shell, gold foil, gold-deposited resin film and metal-deposited resin film. One or more thereof may be used.

Examples of the organic powder may include a silicone rubber powder, a silicone resin-coated silicone rubber powder, polymethylsilsesquioxane, a polyamide powder, a nylon powder, a polyester powder, a polypropylene powder, a polystyrene powder, a polyurethane powder, a vinyl resin powder, a urea resin powder, a phenolic resin powder, a fluorine resin powder, a silicon resin powder, an acrylic resin powder, a melamine resin powder, a polycarbonate resin, a divinylbenzene-styrene copolymer, a silk powder, a wool powder, a cellulose powder, a long-chain alkyl phosphoric acid metal salt, an N-mono long chain alkyl acyl basic amino acid, and a composite thereof. One or more thereof may be used.

It is preferable that of them, a cellulose powder, a silicone rubber powder, a silicone resin-coated silicone rubber powder, polymethylsilsesquioxane, an acrylic resin powder and a nylon powder be included.

All the powders used in the present invention may be directly used, or one or more thereof which have been hydrophobized may also be used. Methods of hydrophobization are not limited as long as they are usually done for a powder for a cosmetic. A dry process or a wet process may be performed using a surface treatment agent such as a fluorine compound, a silicone compound, metal soap, an amino acid compound, lecithin, alkylsilane, an oil agent and organic titanate.

Examples of the surface treatment agent may include fluorine compounds such as perfluoropolyether, perfluoroalkyl phosphate ester, perfluoroalkylalkoxy silane and fluorine-modified silicone; silicone compounds such as dimethylpolysiloxane, methylhydrogenpolysiloxane, cyclic silicone, organopolysiloxane modified with a trialkoxy group at one or both terminals, a crosslinked silicone, a silicone resin, a fluorine-modified silicone resin and acryl-modified silicone; a metal soap such as aluminum stearate, aluminum myristate, zinc stearate and magnesium stearate; amino acid compounds such as proline, hydroxyproline, alanine, glycine, sarcosine, glutamic acid, aspartic acid, lysine and a derivative thereof; lecithin, hydrogenated lecithin; alkyl silane such as methyltrimethoxysilane, ethyltrimethoxysilane, hexyltrimethoxysilane, octyltrimethoxysilane and octyltriethoxysilane; oil agents such as polyisobutylene, wax and oil and fat; and organic titanate such as isopropyltitanium triisostearate.

A powder prepared by hydrophilizing one or more thereof may also be used as the powder used in the present invention. Methods of hydrophilization are not limited as long as they are usually done for a powder for a cosmetic.

Examples thereof may include plant polymers such as gum arabic, tragacanth, arabinogalactan, locust bean gum (carob gum), guar gum, karaya gum, carrageenan, pectin, agar, quince seed (marmelo), starch (rice, corn, potato, wheat), algae colloid, trant gum and locust bean gum; microbial polymers such as xanthan gum, dextran, succinoglucan and pullulan; animal polymers such as collagen, casein, albumin, deoxyribonucleic acid (DNA) and a salt thereof; starch polymers such as carboxymethyl starch and methyl hydroxypropyl starch; cellulose polymers such as methyl cellulose, ethyl cellulose, methyl hydroxypropyl cellulose, carboxymethyl cellulose, hydroxymethyl cellulose, hydroxypropyl cellulose, nitrocellulose, sodium cellulose sulfate, sodium carboxymethyl cellulose, crystalline cellulose and cellulose powder; alginic acid polymers such as sodium alginate and propylene glycol alginate ester; vinyl polymers such as polyvinyl methyl ether, polyvinyl pyrrolidone and carboxyvinyl polymer; polyoxyethylene polymers such as polyethylene glycol and polyethylene glycol silane; polyoxyethylene polyoxypropylene copolymers; acrylic polymers such as sodium polyacrylate, polyethylacrylate and polyacrylic acid amide; and inorganic silicic acid compounds such as silica.

A spherical, a plate-like, a needle-like or an amorphous powder, a fume or a particulate powder, a powder having a particle size of pigment grade, and a porous or a non-porous powder may be used as the powder, as long as they are usually used for cosmetics.

The powder has an average particle size of preferably 0.001 µm or more and 200 µm or less, more preferably 0.01 µm or more and 50 µm or less, further preferably 0.02 µm or more and 20 µm or less, and still more preferably 0.05 µm or more and 10 µm or less in order for the powder to adhere uniformly to the hills, grooves and pores of the skin to create a natural feeling of make-up.

In the present invention, the average particle size of a powder may be measured by a particle size distribution analyzer based on an observation with an electron microscope according to a laser diffraction/scattering method. More specifically, in the laser diffraction/scattering method, measurement is performed by a laser diffraction/scattering particle distribution analyzer (e.g., LMS-350 made by SEISHIN ENTERPRISE Co., Ltd.) using ethanol as a dispersion solvent. When a powder has been hydrophobized or hydrophilized, the average particle size and the content of the component (c) means the average particle size and the mass of a material including the hydrophobizing or hydrophilizing agent.

One or more powders may be used. The content thereof, which varies depending on the form of the cosmetic, is preferably 1% by mass or more, more preferably 3% by mass or more, further preferably 5% by mass or more, and preferably 99% by mass or less, more preferably 95% by mass or less, and further preferably 90% by mass or less in the cosmetic in consideration of finished look. The content of the powder is preferably 1% by mass or more and 99% by mass or less, more preferably 3% by mass or more and 95% by mass or less, and further preferably 5% by mass or more and 90% by mass or less in the cosmetic.

The mass ratio of the color pigment to the whole powder (color pigment/whole powder) is preferably 0.2 or more, more preferably 0.3 or more, and further preferably 0.4 or more, and preferably 1.0 or less in consideration of finished look and excellent durability over time of the cosmetic containing powder with little smudging.

The type of cosmetics used in the Step C) is not particularly limited as long as it is a cosmetic containing a powder. The cosmetic may be used as a cosmetic for makeup, such as a makeup base, a foundation, a concealer, a blush, an eye shadow, a mascara, an eyeliner, an eyebrow, an overcoating agent and a lipstick; and UV protection cosmetics such as a sunscreen emulsion and sunscreen cream. In particular, a makeup base, a foundation, a concealer, a sunscreen emulsion and a sunscreen cream are more preferred.

The form of cosmetics is not particularly limited, and the cosmetic may be any of a powder cosmetic, a solid powder cosmetic, a liquid cosmetic, an oil cosmetic, an emulsion cosmetic and a solid oil cosmetic.

Components included in the cosmetic used in the Step C) other than the powder may include an oil agent (including a liquid oil and a solid oil), an emulsifying agent, a water-soluble polymer, a flavoring agent, a repellent, an antioxidant, a stabilizer, an antiseptic, a thickener, a pH adjuster, a blood circulation promoter, vitamins, a cooling agent, an antiperspirant, a disinfectant, a skin activator and a moisturizer.

In the Step C), the cosmetic may be applied to the skin in an ordinary application manner other than electrostatic spraying in accordance with the type of cosmetics. Examples thereof include spreading and pressing using the fingers or the palm and spreading and pressing using an exclusive tool.

The present invention also provides a composition Y comprising a component (c) and a component (d), the composition Y being used to be applied to skin in a manner other than electrostatic spraying to produce a coating film on the skin before or after forming a coating film on a surface of the skin by direct electrostatic spraying to the skin:
(c) 0.3% by mass or more and 10% by mass or less of a water-soluble polymer;
(d) 40% by mass or more and 95% by mass or less of water.

It is preferable that for the above composition Y, the composition used for electrostatic spraying be a composition X comprising a component (a) and a component (b) :
(a) one or more volatile substances selected from the group consisting of water, an alcohol and a ketone;
(b) a film-forming polymer.

For the above embodiments, the present invention also discloses the following methods and compositions.
<1> A method for producing a coating film on skin, which comprises the steps of:
   A) electrostatically spraying a composition X comprising a component (a) and a component (b) directly to the skin to form a coating film on a surface of the skin:
      (a) one or more volatile substances selected from the group consisting of water, an alcohol and a ketone;
      (b) a film-forming polymer; and
   B) applying a composition Y comprising a component (c) and a component (d) to the skin:
      (c) 0.3% by mass or more and 10% by mass or less of a water-soluble polymer;
      (d) 40% by mass or more and 95% by mass or less of water
   in the order presented or in reverse order.
<2> The method for producing a coating film according to <1>, wherein the coating film formed by electrostatic spraying in the Step A) is a porous coating film.
<3> The method for producing a coating film according to <1> or <2>, wherein the Step A) is a step of forming a coating film comprising a deposit of fiber by electrostatically spraying the composition X to the skin by using an electrostatic spraying apparatus, and the electrostatic spraying apparatus comprises a container for storing the composition X, a nozzle for discharging the composition X, a device for supplying the composition X stored in the container to the nozzle, and a power source for applying voltage to the nozzle.
<4> The method for producing a coating film according to any of <1> to <3>, wherein the volatile substance as the component (a) is preferably one or more selected from the group consisting of ethanol, isopropyl alcohol, butyl alcohol and water, more preferably one or more selected from the group consisting of ethanol and butyl alcohol, and further preferably a volatile substance containing at least ethanol.
<5> The method for producing a coating film according to any of <1> to <4>, wherein a content of the component (a) in the composition X is preferably 30% by mass or more, more preferably 55% by mass or more, and further preferably 60% by mass or more, and preferably 98% by mass or less, more preferably 96% by mass or less, and further preferably 94% by mass or less.
<6> The method for producing a coating film according to any of <1> to <5>, wherein the component (b) is preferably a water-insoluble polymer, more preferably one or more selected from the group consisting of completely saponified polyvinyl alcohol, which can be insolubilized after forming a coating film, partially saponified polyvinyl alcohol, which can be cross-linked after forming a coating film when used in combination with a cross-linking agent, polyvinylbutyral resin, polyurethane resin, an oxazoline-modified silicone such as a poly(N-propanoylethyleneimine)-grafted dimethylsiloxane/y-aminopropylmethylsiloxane copolymer, polyvinylacetal diethylaminoacetate and Zein, and more preferably one or more selected from the group consisting of polybutyral resin and polyurethane resin.
<7> The method for producing a coating film according to any of <1> to <6>, wherein a content of the component (b) in the composition X is preferably 2% by mass or more, more preferably 4% by mass or more, and further preferably 6% by mass or more, and preferably 50% by mass or less, more preferably 45% by mass or less, and further preferably 40% by mass or less.
<8> The method for producing a coating film according to any of <1> to <7>, wherein the component (c) is preferably a polymer with such a characteristic that when 1 g of the polymer is weighed in an environment of 1 atm and 23°C and then dipped in 10 g of ion exchange water for 24 hours, 0.5 g or more of the polymer which has been immersed dissolves in water, and is more preferably one or more selected from the group consisting of a plant polymer, a microbial polymer, an animal polymer, an alginic acid polymer, a mucopolysaccharide, a cellulose polymer, a starch polymer, a vinyl polymer, an acrylic polymer and a polyoxyethylene polymer.
<9> The method for producing a coating film according to any of <1> to <8>, wherein the component (c) is one or more selected from the group consisting of carrageenan, agar, xanthan gum, pullulan, hyaluronic acid, tuberose polysaccharide (TPS), carboxymethyl cellulose, hydroxyethyl cellulose, sodium hydroxyethylcellulose hydroxypropyl stearyl ether hydroxypropylsulfonate, polyvinyl pyrrolidone, carboxyvinyl polymer, (acrylate/alkyl acrylate (C10-30)) crosspolymer, (Na acrylate/acryloyldimethyltaurine Na) copolymer and polyacrylate crosspolymer-6.
<10> The method for producing a coating film according to any of <1> to <9>, wherein a content of the water-soluble polymer (c) in the composition Y is preferably 0.4% by mass or more, more preferably 0.5% by mass or more, and preferably 8% by mass or less, more preferably 7% by mass or less, and further preferably 6% by mass or less.
<11> The method for producing a coating film according to any of <1> to <10>, wherein a content of water (d) in the composition Y is preferably 50% by mass or more, more preferably 60% by mass or more, and further preferably 70% by mass or more, and preferably 94.8% by mass or less, more preferably 94.5% by mass or less, and further preferably 94.0% by mass or less.
<12> The method for producing a coating film according to any of <1> to <11>, wherein a mass ratio of the component (d) to the component (c) in the composition Y, ((d)/(c)), is preferably 10 or more, more preferably 15 or more, and further preferably 20 or more, and preferably 400 or less, more preferably 300 or less, and further preferably 250 or less.
<13> The method for producing a coating film according to any of <1> to <12>, wherein the composition Y further comprises a component (e), a polyol.
<14> The method for producing a coating film according to <13>, wherein the component (e) is preferably ethylene glycol, propylene glycol, 1,3-butanediol, dipropylene glycol, polyethylene glycol having a molecular weight of 1,000 or less, glycerol and diglycerol, and more preferably propylene glycol, 1,3-butanediol and glycerol, and further preferably the component (e) includes at least glycerol.
<15> The method for producing a coating film according to <13> or <14>, wherein a content of the component (e) in the composition Y is preferably 1% by mass or more, more preferably 2% by mass or more, and further preferably 3% by mass, and preferably 30% by mass or less, more preferably 25% by mass or less, and further preferably 20% by mass or less.
<16> The method for producing a coating film according to any of <13> to <15>, wherein a mass ratio of the component (e) to the component (c) in the composition Y, (e/c), is preferably 0.3 or more, more preferably 0.5 or more, and further preferably 1 or more, and preferably 100 or less, more preferably 40 or less, and further preferably 15 or less.
<17> The method for producing a coating film according to any of <1> to <16>, further comprising a step of applying a cosmetic containing a powder to the skin (Step C)) before, between or after the Step A) and the Step B).
<18> A composition Y comprising a component (c) and a component (d), the composition being used to be applied to skin in a manner other than electrostatic spraying to produce a coating film on the skin before or after forming a coating film on a surface of the skin by direct electrostatic spraying to the skin:
   (c) 0.3% by mass or more and 10% by mass or less of a water-soluble polymer;
   (d) 40% by mass or more and 95% by mass or less of water.
<19> The composition Y according to <18>, wherein the composition used for electrostatic spraying is a composition X comprising a component (a) and a component (b) :
   (a) one or more volatile substances selected from the group consisting of water, an alcohol and a ketone;
   (b) a film-forming polymer.

### Examples

Hereinafter the present invention will be described in detail with reference to Examples, but the scope of the present invention is not limited by these Examples. "%" means "% by mass" unless otherwise specified.

### Synthetic Example 1

(1) A 1,000 mL separable glass reactor equipped with a stirrer, a thermometer and a condenser was charged with 50 g of hydroxyethyl cellulose having a weight average molecular weight of 800,000 and a degree of substitution of hydroxyethyl groups of 1.8 (HEC-QP4400 made by Union Carbide Corporation), 400 g of 88% isopropyl alcohol and 3.5 g of a 48% aqueous sodium hydroxide solution to prepare a slurry liquid. The slurry liquid was stirred in a nitrogen atmosphere at room temperature for 30 minutes. 5.4 g of stearyl glycidyl ether was added thereto and the mixture was allowed to react at 80°C for 8 hours to perform hydrophobization. After completion of the hydrophobization reaction, the reaction solution was neutralized with acetic acid and the reaction product was separated by filtration. The reaction product was washed with 500 g of 80% acetone twice and then 500 g of acetone twice, dried under reduced pressure at 70°C for a whole day and night to obtain 49.4 g of a hydrophobized hydroxyethyl cellulose derivative.
(2) A 500 mL separable glass reactor equipped with a stirrer, a thermometer and a condenser was charged with 10.0 g of the hydrophobized hydroxyethyl cellulose derivative obtained in (1), 80.0 g of isopropyl alcohol and 0.33 g of a 48% aqueous sodium hydroxide solution to prepare a slurry liquid. The slurry liquid was stirred in a nitrogen stream at room temperature for 30 minutes. A mixture prepared by mixing 6.4 g of sodium 3-chloro-2-hydroxypropanesulfonate, 2.7 g of a 48% aqueous sodium hydroxide solution and 20.0 g of water was added to the reaction solution to perform sulfonation at 50°C for 9 hours. After completion of the reaction, the reaction solution was neutralized with acetic acid and the product was separated by filtration. The resulting product was washed with 500 g of 80% acetone (20% water) three times and 500 g of acetone twice, and then dried under reduced pressure at 70°C for a whole day and night to obtain 7.2 g of a water-soluble alkyl-substituted polysaccharide derivative (1) substituted by a stearyl glyceryl ether group and a sulfo-2-hydroxypropyl group.

In the resulting water-soluble alkyl-substituted polysaccharide derivative (1), the degree of substitution by the stearyl glyceryl ether group was 0.030 and the degree of substitution by the sulfo-2-hydroxypropyl group was 0.15. The ratio of the number of hydrophobic moiety substituents (a) to the number of hydrophilic moiety substituents (b) was 30 : 150.

### [Test 1]

(1) Preparation of spraying composition
   The composition of Table 1 was used as a spraying composition.
(2) Preparation of composition Y
   The liquid formulations (composition Y) shown in Table 2 were used.
(3) Process of evaluation
   I. The composition Y was applied to the skin (Step B)). 100 µL of the composition Y was dropped on the back of a person's hand with a micropipette, and spread with the finger in an area with a diameter of 4 cm or more and less than 6 cm to sink the composition thereinto and form a thin layer.
   II. Electrostatic spraying was performed (Step A))
      An electrostatic spraying method was performed in an area to which a composition of artificial leather had been applied for 60 seconds using the electrostatic spraying apparatus 10 having the structure shown in Fig. 1 and the appearance shown in Fig. 2. The conditions of the electrostatic spraying method were as shown below.
      - Voltage applied: 10 kV
      - Distance between nozzle and skin: 100 mm
      - Discharged amount of spraying composition: 5 mL/h
      - Environment: 25°C, 50%RH

      A porous coating film made of a deposit of fiber was formed on the back of the hand by the above electrostatic spraying. The composition was applied thereto in the form of a circle having a diameter of 4 cm or more and less than 6 cm.
   III. The coating film was lightly tapped with the finger, sponge and the like to sink the coating film into the composition Y.

### [Test 2]

The order of the step of applying a liquid formulation and the step of electrostatic spraying in Test 1 was reversed. A porous coating film made of a deposit of fiber was prepared in the same manner as in Test 1 except for the above.

Then the coating film formed on the back of the hand was subjected to sensory evaluation based on the following criteria.

### [Evaluation 1] Scratch resistance

The adhesiveness to the skin of the coating films formed in Examples and Comparative Examples was evaluated. In the evaluation, the coating film was touched by the finger in the direction perpendicular to the skin to apply a micro-vibration load, and the finger was moved back and forth in the direction parallel to the skin to apply shear force to the coating film, and the state of the coating film thereafter was visually observed. The results are shown in Table 1. The criteria of evaluation are as follows.
1. Substantially all of the coating film peels off when a micro-vibration load is applied thereto by the finger in the perpendicular direction.
2. Part of the fiber constituting the coating film peels off when a micro-vibration load is applied thereto by the finger in the perpendicular direction.
3. No peeling occurs in the perpendicular direction, but substantially all of the coating film peels off when shear force is applied thereto in the parallel direction.
4. No peeling occurs in the perpendicular direction, but part of the coating film or the fiber peels off when shear force is applied thereto by the finger in the parallel direction.
5. No peeling occurs in the perpendicular direction and the coating film or the fiber does not peel off even when shear force is applied thereto in the parallel direction.

### [Test 3]

(1) Preparation of spraying composition
   The composition of Table 1 was used as a spraying composition.
(2) Preparation of composition Y
   The liquid formulations (composition Y) shown in Table 2 were used.
(3) Process of evaluation
   I. The composition Y was applied to artificial leather having a thickness of 0.7 mm (Protein leather PBZ13001BK made by Idemitsu Technofine Co., Ltd.) (Step B)). 50 mg of the composition Y was applied to the artificial leather in an area of 5 cm × 5 cm.
   II. Electrostatic spraying was performed (Step A))
      An electrostatic spraying method was performed in an area to which a composition of artificial leather had been applied for 60 seconds using the electrostatic spraying apparatus 10 having the structure shown in Fig. 1 and the appearance shown in Fig. 2. The conditions of the electrostatic spraying method were as shown below.
      - Voltage applied: 10 kV
      - Distance between nozzle and skin: 100 mm
      - Discharged amount of spraying composition: 5 mL/h
      - Environment: 25°C, 50%RH

      A porous coating film made of a deposit of fiber was formed on the surface of the artificial leather by the above electrostatic spraying. The composition was applied thereto in the form of a square of 7 cm × 7 cm.
   III. The coating film was lightly tapped with the finger, sponge and the like to sink the coating film into the composition Y, and then dried at room temperature overnight.

Then, the coating film formed on the artificial leather was evaluated based on the following criteria.

### [Evaluation 2] Appearance

An image of the coating film formed (artificial leather) was taken by a scanner (GTX830 made by EPSON, 24 bit, 600 dpi), and the average of gray values (0 to 255) of the area to which the liquid formulation was applied was calculated by Image J.

### [Evaluation 3] Stretch resistance

The side having a length of 10 cm of the coating film formed (artificial leather) was stretched by the hand to 12 cm, and this was repeated 10 times, and then the coating film was evaluated as to whether wrinkles, float or cracks occur on the coating film.

Evaluation criteria:
1: Wrinkles, float and cracks occur in 75% or more of the area of the coating film to which the composition Y was applied after the 10 tensile tests.
2: Wrinkles, float and cracks occur in 50% or more and less than 75% of the area of the coating film to which the composition Y was applied after the 10 tensile tests.
3: Wrinkles, float and cracks occur in 25% or more and less than 50% of the area of the coating film to which the composition Y was applied after the 10 tensile tests.
4: Wrinkles, float and cracks occur in less than 25% of the area of the coating film to which the composition Y was applied after the 10 tensile tests.
5: The appearance of the area of the coating film to which the composition Y was applied did not change after the 10 tensile tests.

### [Evaluation 4] Evaluation of touch

Five reviewers touched the coating film formed (artificial leather) by the finger and evaluated the absence of oily feeling on a scale of 1 (significant oily feeling) to 5 (little oily feeling). The average of the scores of the five reviewers was calculated.

**[Table 1]**

| Component | | Spraying Composition 1 |
|---|---|---|
| (a) | Ethanol | 87.56 |
| | Water | 0.44 |
| (b) | Polyvinylbutyral*1 | 12.00 |
| Total | | 100.00 |

| | | |
|---|---|---|
| ***1: S-LEC B BM-1 (SEKISUI CHEMICAL CO.,LTD.)** | | |

**[Table 2]**

| | | Liquid formulation | Liquid formulation | Liquid formulation | Liquid formulation | Liquid formulation | Liquid formulation | Liquid formulation | Liquid formulation | Liquid formulation | Liquid formulation | Liquid formulation | Liquid formulation |
|---|---|---|---|---|---|---|---|---|---|---|---|---|---|
| | Component (% by mass) | 1 | 2 | 3 | 4 | 5 | 6 | 7 | 8 | 9 | 10 | 11 | 12 |
| | Carrageenan*1 | 1.00 | | | | | | | | | | | |
| | Xanthan gum*2 | | 0.40 | | | | | | | | | | |
| | Pullulan*3 | | | 0.50 | 1.00 | 3.00 | | | | | | | |
| | Hyaluronic acid*4 | | | | | | 0.50 | | | | | | |
| | Tuberose polysaccharide*5 | | | | | | | 1.00 | | | | | |
| | Carboxymethyl cellulose*6 | | | | | | | | 1.00 | 2.00 | 3.00 | | |
| | Hydroxyethyl cellulose*7 | | | | | | | | | | | 1.00 | 2.00 |
| | Sodium hydroxyethylcellulose hydroxypropyl stearyl ether hydroxypropylsulfonate Synthetic Example 1 | | | | | | | | | | | | |
| (c) | Polyvinyl pyrrolidone*8 | | | | | | | | | | | | |
| | Carboxyvinyl polymer*9 | | | | | | | | | | | | |
| | (Acrylate/alkyl acrylate (C10-30)) crosspolymer*10 | | | | | | | | | | | | |
| | (Acrylate/alkyl acrylate (C10-30)) crosspolymer*11 | | | | | | | | | | | | |
| | (Na acrylate/acryloyldimeth yltaurine Na) copolymer*12 | | | | | | | | | | | | |
| | Polyacrylate crosspolymer-6* 13 | | | | | | | | | | | | |
| (d) | Water | 93.19 | 93.79 | 93.69 | 93.19 | 91.19 | 93.69 | 92.45 | 93.19 | 92.19 | 91.19 | 93.19 | 92.19 |
| (d)+(e) | Glycerol (86%) + water (14%) | 5.81 | 5.81 | 5.81 | 5.81 | 5.81 | 5.81 | | 5.81 | 5.81 | 5.81 | 5.81 | 5.81 |
| Other compon ents | Squalane | | | | | | | | | | | | |
| | Ethanol | | | | | | | 6.45 | | | | | |
| | Methyl paraoxybenzoate | | | | | | | 0.10 | | | | | |
| | Isohexadecane | | | | | | | | | | | | |
| | Polyoxyethylene (20) sorbitan monooleate | | | | | | | | | | | | |
| | Sorbitan monooleate | | | | | | | | | | | | |
| | Total | 100.00 | 100.00 | 100.00 | 100.00 | 100.00 | 100.00 | 100.00 | 100.00 | 100.00 | 100.00 | 100.00 | 100.00 |
| | (c)Solid content of water-soluble polymer | 1.0 | 0.4 | 0.5 | 1.0 | 3.0 | 0.5 | 1.0 | 1.0 | 2.0 | 3.0 | 1.0 | 2.0 |
| | (d)Water | 94.0 | 94.6 | 94.5 | 94.0 | 92.0 | 94.5 | 92.5 | 94.0 | 93.0 | 92.0 | 94.0 | 93.0 |
| | (e) | 5.0 | 5.0 | 5.0 | 5.0 | 5.0 | 5.0 | 0.0 | 5.0 | 5.0 | 5.0 | 5.0 | 5.0 |
| | (d)/(c) | 94 | 237 | 189 | 94 | 31 | 189 | 92 | 94 | 47 | 31 | 94 | 47 |
| | (e)/(c) | 5.0 | 12.5 | 10.0 | 5.0 | 1.7 | 10.0 | - | 5.0 | 2.5 | 1.7 | 5.0 | 2.5 |

| | | | | | | | | | | | | | |
|---|---|---|---|---|---|---|---|---|---|---|---|---|---|
| *1) Carrageenan CS-67 made by San-Ei Gen F.F.I., Inc. *2) Eco gum T made by DSP GOKYO FOOD & CHEMICAL Co., Ltd. *3) Cosmetic Grade Pullulan made by Hayashihara Co., Ltd. *4) Sodium hyaluronate *5) SOFCARE TP-S made by Kao Corporation *6) CELLOGEN F5A made by DKS Co., Ltd. *7) HEC DAICEL SE400 made by DAICEL FINECHEM Ltd. *8) Luviskol K90 made by BASF *9) Carbopol 980 made by Lubrizol Advanced Materials *10) Carbopol ETD2020 made by Lubrizol Advanced Materials *11) Pemulen TR-1 made by Lubrizol Advanced Materials *12) SIMULGEL EG made by SEPPIC *13) SEPIMAX ZEN made by SEPPIC | | | | | | | | | | | | | |

**[Table 3]**

| | | Liquid formulation | Liquid formulation | Liquid formulation | Liquid formulation | Liquid formulation | Liquid formulation | Liquid formulation | Liquid formulation | Liquid formulation | Liquid formulation | Liquid formulation | Liquid formulation |
|---|---|---|---|---|---|---|---|---|---|---|---|---|---|
| | Component (% by mass) | 13 | 14 | 15 | 16 | 17 | 18 | 19 | 20 | 21 | 22 | 23 | 24 |
| | Carrageenan *1 | | | | | | | | | | | | |
| | Xanthan gum*2 | | | | | | | | | | | | |
| | Pullulan*3 | | | | | | | | | | | | |
| | Hyaluronic acid*4 | | | | | | | | | | | | |
| | Tuberose polysaccharid e*5 | | | | | | | | | | | | |
| | Carboxymethylcellulose*6 | | | | | | | | | | | | |
| | Hydroxyethyl cellulose*7 | 3.00 | | | | | | | | | | | |
| | Sodium hydroxyethylcellulose hydroxypropylstearyl ether hydroxypropylsulfonate Synthetic Example 1 | | 1.00 | | | | | | | | | | |
| (c) | Polyvinyl pyrrolidone*8 | | | 1.00 | 2.00 | 3.00 | | | | | | | |
| | Carboxyvinyl polymer*9 | | | | | | 1.00 | | | | | | |
| | (Acrylate/alkylacrylate (C10-30)) crosspolymer *10 | | | | | | | 1.00 | | | | | |
| | (Acrylate/alkylacrylate (C10-30)) crosspolymer *11 | | | | | | | | 1.00 | | | | |
| | (Naacrylatelacryloyldimethyltaurine Na) copolymer*12 | | | | | | | | | 3.75 | | | |
| | Polyacrylate crosspolymer -6*13 | | | | | | | | | | 1.00 | | |
| (d) | Water | 91.19 | 93.19 | 93.19 | 92.19 | 91.19 | 93.19 | 93.19 | 93.19 | 93.00 | 93.19 | 100 | 94.19 |
| (d)+( e) | Glycerol (86%) + water (14%) | 5.81 | 5.81 | 5.81 | 5.81 | 5.81 | 5.81 | 5.81 | 5.81 | | 5.81 | | 5.81 |
| Other components | Squalane | | | | | | | | | | | | |
| | Ethanol | | | | | | | | | | | | |
| | Methylparaoxybenzoate | | | | | | | | | | | | |
| | Isohexadecane | | | | | | | | | 2.25 | | | |
| | Polyoxyethylene (20) sorbitan monooleate | | | | | | | | | 0.75 | | | |
| | Sorbitan monooleate | | | | | | | | | 0.25 | | | |
| | Total | 100.00 | 100.00 | 100.00 | 100.00 | 100.00 | 100.00 | 100.00 | 100.00 | 100.00 | 100.00 | 100.00 | 100.00 |
| | (c)Solid content of water-soluble polymer | 3.0 | 1.0 | 1.0 | 2.0 | 3.0 | 1.0 | 1.0 | 1.0 | 3.8 | 1.0 | - | - |
| | (d)Water | 92.0 | 94.0 | 94.0 | 93.0 | 92.0 | 94.0 | 94.0 | 94.0 | 93.0 | 94.0 | 100.0 | 95.0 |
| | (e) | 5.0 | 5.0 | 5.0 | 5.0 | 5.0 | 5.0 | 5.0 | 5.0 | 0.0 | 5.0 | - | 5.0 |
| | (d)/(c) | 31 | 94 | 94 | 47 | 31 | 94 | 94 | 94 | 25 | 94 | - | - |
| | (e)/(c) | 1.7 | 5.0 | 5.0 | 2.5 | 1.7 | 5.0 | 5.0 | 5.0 | - | 5.0 | - | |

**[Table 4]**

| | | Example 1 | Example 2 | Example 3 | Example 4 | Example 5 | Example 6 | Example 7 | Example 8 | Example 9 | Example 10 | Example 11 | Example 12 | Example 13 |
|---|---|---|---|---|---|---|---|---|---|---|---|---|---|---|
| I | Application order1 | Liquid formulation 1 | Liquid formulation 2 | Liquid formulation 3 | Liquid formulation 4 | Liquid formulation 5 | Liquid formulation 6 | Liquid formulation 7 | Liquid formulation 8 | Liquid formulation 9 | Liquid formulation 10 | Liquid formulation 11 | Liquid formulation 12 | Liquid formulation 13 |
| II | Application order 2 | Spraying compositio n 1 | Spraying compositio n 1 | Spraying compositio n 1 | Spraying compositio n 1 | Spraying compositio n 1 | Spraying compositio n 1 | Spraying compositio n 1 | Spraying compositio n 1 | Spraying compositio n 1 | Spraying compositio n 1 | Spraying compositio n 1 | Spraying compositio n 1 | Spraying compositio n 1 |
| Evaluatio n | Scratch resistance (1. Poor to 5. Good) | 4 | 5 | 4 | 4 | 5 | 4 | 4 | 2 | 4 | 5 | 4 | 4 | 5 |
| | Appearance (gray value) | 133 | 148 | 146 | 145 | 126 | 128 | 122 | 130 | 133 | 122 | 136 | 129 | 114 |
| | Stretch resistance (1. Poor to 5. Good) | 5 | 5 | 4 | 5 | 5 | 2 | 2 | 2 | 4 | 5 | 3 | 2 | 2 |
| | Absence of oily feeling (1. Oily to 5. Not oily) | 5 | 5 | 5 | 4.8 | 5 | 5 | 5 | 5 | 5 | 5 | 5 | 5 | 5 |

**[Table 5]**

| | | Example 14 | Example 15 | Example 16 | Example 17 | Example 18 | Example 19 | Example 20 | Example 21 | Example 22 | Comparative Example 1 | Example 2 |
|---|---|---|---|---|---|---|---|---|---|---|---|---|
| I | Application order 1 | Liquid formulation 14 | Liquid formulation 15 | Liquid formulation 16 | Liquid formulation 17 | Liquid formulation 18 | Liquid formulation 19 | Liquid formulation 20 | Liquid formulation 21 | Liquid formulation 22 | Liquid formulation 23 | Liquid formulation 24 |
| II | Application order 2 | Spraying composition 1 | Spraying composition 1 | Spraying composition 1 | Spraying composition 1 | Spraying composition 1 | Spraying composition 1 | Spraying composition 1 | Spraying composition 1 | Spraying composition 1 | Spraying composition 1 | Spraying composition 1 |
| Evaluation | Scratch resistance (1. Poor to 5. Good) | 4 | 4 | 5 | 5 | 4 | 2 | 4 | 4 | 4 | 2 | 2 |
| | Appearance (gray value) | 136 | 138 | 124 | 122 | 132 | 133 | 103 | 79 | 115 | 149 | 166 |
| | Stretch resistance (1. Poor to 5. Good) | 5 | 5 | 2 | 3 | 2 | 5 | 5 | 3 | 3 | 1 | 1 |
| | Absence of oily feeling (1. Oily to 5. Not oily) | 5 | 5 | 5 | 5 | 5 | 4.8 | 5 | 5 | 5 | 4.6 | 4.8 |

**[Table 6]**

| | | Example 2 | Example 23 | Comparative Example 1 | Example 2 |
|---|---|---|---|---|---|
| I | Application order 1 | Liquid formulation 2 | Spraying composition 1 | Liquid formulation 23 | Liquid formulation 24 |
| II | Application order 2 | Spraying composition 1 | Liquid formulation 2 | Spraying composition 1 | Spraying composition 1 |
| Evaluation | Scratch resistance (1. Poor to 5. Good) | 5 | 4 | 2 | 2 |

Table 2 to Table 6 show that it is necessary that the composition Y contain (c) a water-soluble polymer and (d) water.

### Reference Signs List

- 10: Electrostatic spraying apparatus
- 11: Low-voltage power source
- 12: High-voltage power source
- 13: Auxiliary electronic circuit
- 14: Pump mechanism
- 15: Container
- 16: Nozzle
- 17: Pipe
- 18: Flexible Pipe
- 19: Current limiting resistor
- 20: Housing

## Claims

1. A method for producing a coating film on skin, comprising the steps of:
A) electrostatically spraying a composition X comprising a component (a) and a component (b) directly to the skin to form a coating film on a surface of the skin:
(a) one or more volatile substances selected from the group consisting of water, an alcohol and a ketone;
(b) a film-forming polymer; and
B) applying a composition Y comprising a component (c) and a component (d) to the skin:
(c) 0.3% by mass or more and 10% by mass or less of a water-soluble polymer;
(d) 40% by mass or more and 95% by mass or less of water
in the order presented or in reverse order.

2. The method for producing a coating film according to claim 1, wherein a mass ratio of the component (d) to the component (c) in the composition Y, ((d)/(c)), is 10 or more and 400 or less.

3. The method for producing a coating film according to claim 1 or 2, wherein the composition Y further comprises (e) a polyol.

4. The method for producing a coating film according to claim 3, wherein a content of the component (e) in the composition Y is 1% by mass or more and 30% by mass or less.

5. The method for producing a coating film according to claim 3 or 4, wherein a mass ratio of the component (e) to the component (c) in the composition Y, ((e)/(c)), is 0.3 or more and 200 or less.

6. The method for producing a coating film according to any one of claims 1 to 5, wherein the coating film formed by the electrostatic spraying in the Step A) is a porous coating film.

7. The method for producing a coating film according to any one of claims 1 to 6, wherein the Step A) is a step of forming a coating film comprising a deposit of fiber by electrostatically spraying the composition X to the skin by using an electrostatic spraying apparatus, and the electrostatic spraying apparatus comprises a container for storing the composition X, a nozzle for discharging the composition X, an apparatus for supplying the composition X stored in the container to the nozzle, and a power source for applying voltage to the nozzle.

8. A composition Y comprising a component (c) and a component (d), the composition Y being used to be applied to skin in a manner other than electrostatic spraying to produce a coating film on the skin before or after forming a coating film on a surface of the skin by direct electrostatic spraying to the skin:
(c) 0.3% by mass or more and 10% by mass or less of a water-soluble polymer;
(d) 40% by mass or more and 95% by mass or less of water.

9. The composition Y according to claim 8, wherein the composition used for electrostatic spraying is a composition X comprising a component (a) and a component (b) :
(a) one or more volatile substances selected from the group consisting of water, an alcohol and a ketone;
(b) a film-forming polymer.
